# EUROPEAN PATENT SPECIFICATION

(11) **EP 0 923 527 B1**
(45) Date of publication and mention of the grant of the patent: **16.01.2002**
(21) Application number: 97941895.1
(22) Date of filing: 25.07.1997
(51) Int. Cl.: C07C 57/30, C07C 59/68, C07C 59/84, C07C 51/16, C07C 309/00, C07C 49/76, C07C 211/43

(54) **A PROCESS FOR THE PREPARATION OF 2-(3-BENZOYL-PHENYL)-PROPIONIC ACID STARTING FROM ARYL-OLEFINS**
VERFAHREN ZUR HERSTELLUNG VON 2-(3-BENZOYL-PHENYL)-PROPIONSÄURE AUS ARYLOLEFINEN
PROCEDE POUR PREPARER L'ACIDE 2-(3-BENZOYL-PHENYL)-PROPIONIQUE A PARTIR D'ARYL-OLEFINES

(30) Priority: 02.08.1996 IT MI961683
(43) Date of publication of application: 23.06.1999
(73) Proprietor: Dompé S.P.A., I-67100 L'Aquila (IT)
(72) Inventor: MANTOVANINI, Marco, I-67100 L'Aquila (IT); ALLEGRETTI, Marcello, I-67100 L'Aquila (IT); CLAVENNA, Gaetano, I-67100 L'Aquila (IT); GANDOLFI, Carmelo, I-67100 L'Aquila (IT)
(74) Representative: Minoja, Fabrizio, Dr.
(86) International application number: EP9704050
(87) International publication number: WO9805623

(56) References cited:
- EP-A- 0 394 949
- GB-A- 1 586 798
- GB-A- 2 025 397

## Description

The present invention relates to a process for the preparation of Ketoprofen.

Ketoprofen is one of the most widely used non-steroidal antiinflammatory agents, with a consumption in the region of hundreds of thousands tons a year.

The available, more established synthetic procedures involve usually reagents (bromine, cyanides, benzene) and/or carboxylation and alkylation reactions which, although formerly usual, are now undesirable in terms of safety of the workers as well as environmental impact, which is nowadays an exceedingly serious problem.

GB 1586798 describes a process for the preparation of phenyl- and naphtyl-alkanoic acids based on the oxidation of an alkene derivative using various oxidating agents.

EP 394949 discloses a process for producing allylsubstituted phenol compounds, and poly(hydroxyphenyl) compounds obtained by such a process.

GB 2025397 describes a process for the preparation of arylacetic acids based on the catalytic hydrogenation of a substituted phenol derivative.

Now a process has been found for the preparation of 2-(3-benzoyl-phenyl)-propionic acid (Ketoprofen), which comprises:
a) Claisen rearrangement of compounds of formula (I): 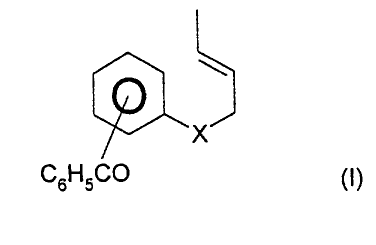 in which X is O, S or NH, the benzoyl group is at the ortho or para position to the X-alkylene group, provided that where X is S, the reaction is carried out in the presence of C3-C10 fatty acid anhydrides, to give a compound of formula (II) 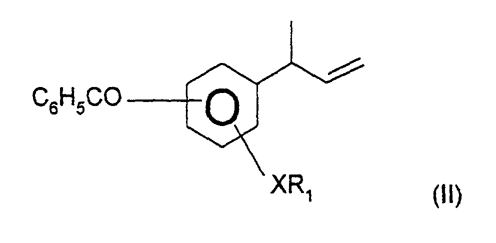 wherein XR₁ is at the ortho position to the allyl chain and is a OH, NH₂, SH or S(C₃-C₁₀) acyl group;
b) for compounds where X is S, desulfuration with Raney nickel,
   for compounds where X is NH, optional deamination via nitrosation with alkali nitrites followed by decomposition of the diazonium salt in the presence of copper sulfate catalytic amounts,
   for compounds where X is O, optional dehydroxylation by forming a trifluoromesylate which is then treated with formic acid and triethylamine in the presence of palladium acetate/triphenylphosphine complex;
c) oxidative cleavage of the compound (II);
d) dehydroxylation or deamination of the product obtained from (c), when such reactions are not already carried out in step b). Claisen rearrangement of the compounds of formula I in step a) is effected using conventional conditions, for example using solvents such as hydrocarbons, aromatic hydrocarbons, halo hydrocarbons or mixtures thereof, at a temperature ranging from 50°C to the solvent's reflux temperature, for times from 2 to 10 hours.

The compounds of formula I are known or they can be prepared according to known methods, starting from suitable phenols, anilines or thiophenols, by reaction with suitable allyl or crotyl halides, in the presence of strong bases and of solvents such as halo hydrocarbons, ethers, aromatic hydrocarbons or mixtures thereof.

The OH or NH₂ groups resulting from the Claisen rearrangement at the ortho position to the allyl chain can be removed with conventional methods: in case of amino groups, nitrosation reactions with alkali nitrites will for example be used, followed by decomposition of the diazonium salt in the presence of copper sulfate catalytic amounts. The OH groups, on the other hand, can be removed treating the corresponding trifluoromesylate with formic acid and triethylamine in the presence of palladium acetate/triphenylphosphine complex. Said reactions can be indifferently carried out before or after the double bond degradation reaction to give the carboxylic acid (step c).

The oxidative cleavage of the double bond can be carried out either by means of ozonolysis or using potassium permanganate in phase transfer conditions. During the oxidative cleavage of the double bond in phase transfer conditions with KMnO₄, the benzyl group linked to the aromatic ring is oxidized at the same time into the benzoyl group.

When the XR₁ group is an S-acyl group, known desulfuration reactions can be effected, for example by means of Raney nickel, but such reactions have to be carried out before the oxidative cleavage of the double bond. The desulfuration reaction can be carried out directly on the S-acyl group, or on the SH group, resulting from the hydrolysis of the S-acyl group.

Claisen rearrangement of the compounds of formula I in which X is sulfur takes place in the presence of C₃-C₁₀ fatty acid anhydrides, for example butyric anhydride or higher ones, as described in Tetrahedron Letters 1971, 1969, to give directly the compounds of formula II wherein R₁ is an acyl group having 3 to 10 carbon atoms.

The process of the invention is particularly advantageous from the environmental point of view, allows to obtain the products in very high yields and provides a versatile synthetic procedure.

The following examples further illustrate the invention.

### EXAMPLE 1

### Preparation of Ketoprofen

### a) from 4-hydroxy-benzofenone

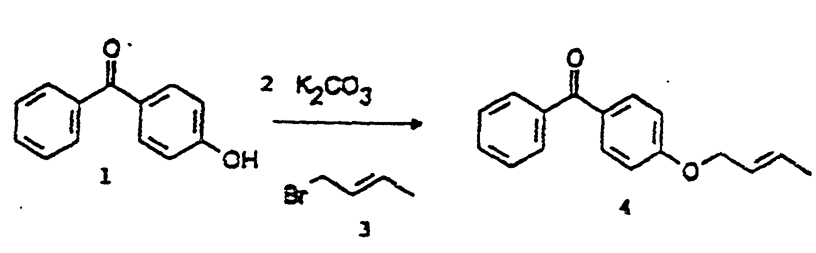

Compound **1** (3 g, 15.1 mmoles) was dissolved in 30 ml of acetone, **2** (4.2 g, 30 mmoles) was added keeping the mixture under stirring. Compound **3** (1.6 ml, 15.5 mmoles) dissolved in 15 ml of acetone was dropped into the mixture, which was kept under stirring at room temperature for 12 hours and subsequently at 40°C for 3 hours. The reaction was complete (TLC control Hexane/EtOAc 8:2). Acetone was evaporated off, the residue was taken up with water (20 ml) and extracted with EtOAc (50 ml). The organic extracts were washed with brine and dried over Na₂SO₄, then filtered and the solvent was evaporated off. 3.8 g (15 mmoles) of the product were recovered (pale yellow oil, solid at + 4°C) sufficiently pure to be used in the subsequent reaction. Hexane/EtOAc 7:3. Rf = 0.38. 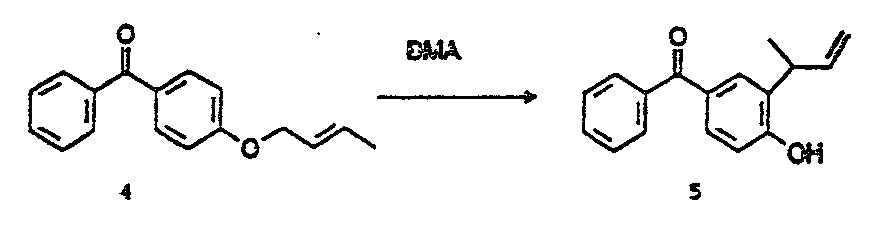 2.8 g of compound **4** (11.1 mmoles) were dissolved in DMA (dimethylaniline), heating to a temperature of 210° (temperature of the outer oil bath) for 10 hours (2 x 5 hours). The progress of the reaction was controlled by TLC (Hexane/Etoac 7:3) on a sample taken from the mixture and poured into a 2N HCl solution and extracted with EtOAc.
At the end of 10 hours the starting product had almost disappeared; the mixture was worked up as the control samples. The organic extracts were washed first with 2N HCl to remove completely DMA, then with brine and dried over Na₂SO₄, filtered and the solvent was evaporated off. A crude weighing 2.55 g was obtained, which was purified on a chromatographic column (Hexane/EtOAc 8:2). The resulting product had still a slightly yellow colour which disappeared upon washing with Hexane:EtOAc 7:3. 1.97 g (7.8 mmoles) of a white solid **5** were obtained. Hexane/EtOAc 7:3. Rf = 0.14. 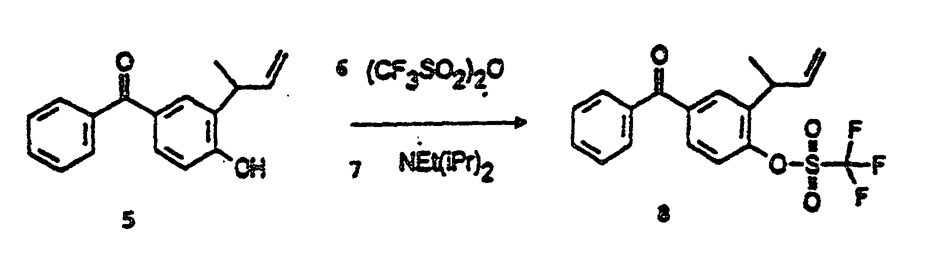 Compound **5** (1.8 g 7.15 mmoles) was dissolved in 18 ml of dry CH₂Cl₂ under argon atmosphere. The mixture was cooled to a temperature of -25°C with an acetone/dry ice mixture. Compound **7** (1.27 ml, 7.3 mmoles) was added, stirring for 30 minutes, then **6** (1.17 ml, 7.15 mmoles) was added in small portions, checking the temperature. The mixture was kept under stirring at -25°C for a further hour, then it was left to warm at room temperature, diluted with ethyl ether and washed with 2N HCl (3 x 10 ml) and with brine (2 x 10 ml). The mixture was then dried over Na₂SO₄, filtered and the solvent was evaporated off. 2.5 g of a pale yellow oil **8** were obtained, which was used in the subsequent reaction.

Hexane/EtOAc 95:5, Rf = 0.59 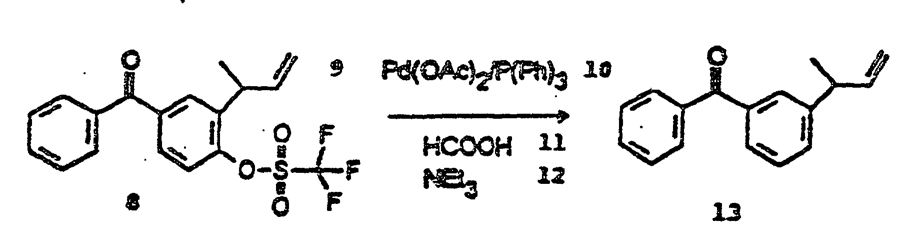 Compound **8** (2.5 g, 6.52 mmoles) was dissolved in 18 ml of DMF, **12** (19.4 ml, 2.7 ml) was added at room temperature and subsequently compounds **9** (57 mg, 0.255 mmoles) and **10** (134 mg, 0.511 mmoles) were added under an argon stream. After that, compound **11** (0.6 g, 13.1 moles) was added in small amounts. Temperature was raised to 60°C for 1 hour. The solution changed colour to black. An amount of the mixture was taken with a capillary tube for the control, the content was shaken in 2N HCl and extracted with CH₂Cl₂. The TLC control showed the completion of the reaction. The cooled mixture was poured into 2N HCl and extracted repeatedly with ethyl ether. The organic phase was washed with 2N HCl, sat. NaHCO₃ and brine, then dried over Na₂SO₄ and the solvent was evaporated off. Compound **13** was obtained as a yellow oil (1.45 g, 6.2 mmoles). Hexane/EtOAc 95:5 - Rf = 0.65. 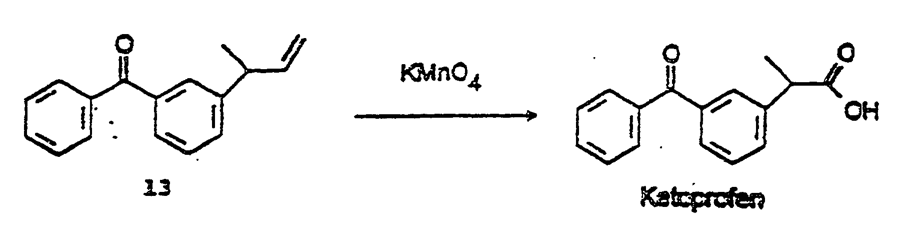 Compound **13** (1.4 g, 5.93 mmoles) was dissolved in CH₂Cl₂ (35 ml), then water (35 ml), conc. H₂SO₄ (1.89 ml), glacial acetic acid (0.7 ml) and a small amount of Aliquat were added. The mixture was stirred vigorously at room temperature and KMnO₄ (2.7 g, 17 mmoles) was added in small amounts, stirring for 48 hours. A TLC control of the organic phase evidenced the disappearance of the starting product. The mixture was added with Na₂S₂O₅ until the brown colour due to manganese dioxide disappears completely, then it was diluted with CH₂Cl₂ and the phases were separated. The organic phase was extracted with sat. NaHCO₃, the aqueous phase was acidified with 2N HCl and extracted with EtOAc. The organic phase was dried over Na₂SO₄ and evaporated. The crude (950 mg) was taken up into a benzene/petroleum ether 6:20 mixture to crystallize pure Ketoprofen (white solid) (725 mg, 2.85 mmoles). CH₂Cl₂/CH₃OH 95:5 Rf = 0.14.

### EXAMPLE 2

### Preparation of Ketoprofen (see example 1)

### b) from 4-nitro-benzofenone

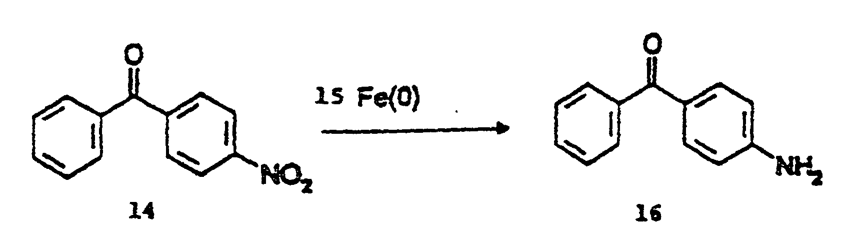

Compound **15** (13 g, 0.23 moles) was suspended in 50 ml of H₂O, 8 drops of conc. HCl were added and the mixture was heated to ebullition. After 1/2 hour compound **14** (10.4 g, 0.045 moles) dissolved in 50 ml of 95% EtOH was dropped therein. The mixture was heated again to ebullition, keeping reflux for 2 hours. A TLC control (Hexane/EtOAc 7:3) showed the reaction was complete. EtOH was evaporated off, the mixture was alkalinized with 2N NaOH, extracted with EtOAc (2x100 ml), dried over Na₂SO₄ and the solvent was evaporated off. A pale yellow solid was obtained, which was washed with an hexane/EtOAc mixture.
Compound **16** was obtained as a white solid (7.1 g, 0.036). Hexane/EtOAc 7:3 Rf = 0.3. 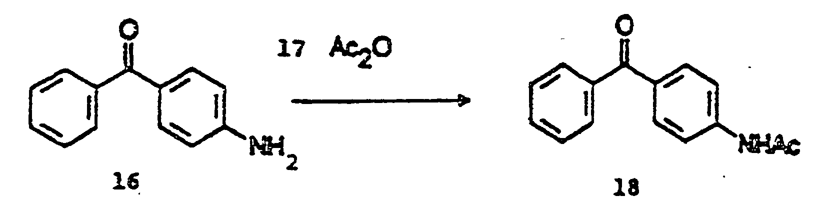 Compound **16** (7.1 g, 0.036 moles) was dissolved in dry CH₂Cl₂ (50 ml), **17** (3.46 ml, 0.036 moles) was added at room temperature, stirring for 12 hours. A TLC control (Hexane: EtOAc 7:3) evidenced the disappearance of the starting product. Solvent was evaporated off and the formed acetic acid was removed by evaporation (azeotropic mixture with toluene). 8.3 g (0.035 moles) of **18** as a white solid were obtained.
Hexane/EtOAc 7:3 Rf = 0.1. 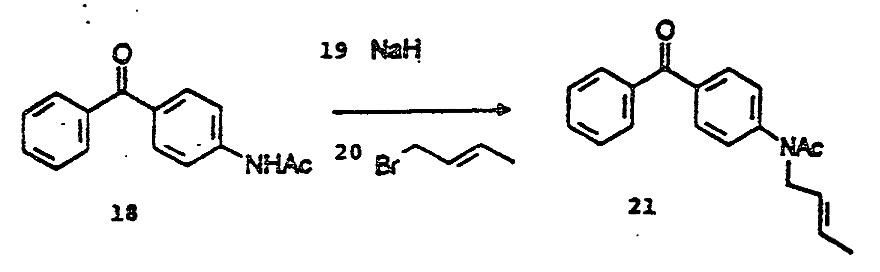 Compound **18** (8.2 g, 0.035 moles) was dissolved in hot CH₂Cl₂ (80 ml) (50°C), the solution was left to cool slightly and **19** (2.1 g of a 60% mineral oil dispersion, 0.052 moles) was added. The mixture was heated to 50°C, stirring for 30 minutes. The abundant precipitate formed was added with compound **20** (4.23 ml, 0.042 moles) dissolved in 20 ml of dry CH₂Cl₂. The mixture was kept overnight at room temperature and at 50°C for 8 hours. The starting product had almost disappeared, the mixture was diluted with CH₂Cl₂, washed with a 5% NaH₂PO₄ solution, then with brine. The organic phase was dried over Na₂SO₄ and the solvent was evaporated off. Compound **21** was obtained as a pale yellow solid, which was sufficiently pure to be used in the subsequent reaction (8.6 g 0,029 moles). Hexane/EtOAc 7:3 Rf = 0.44. 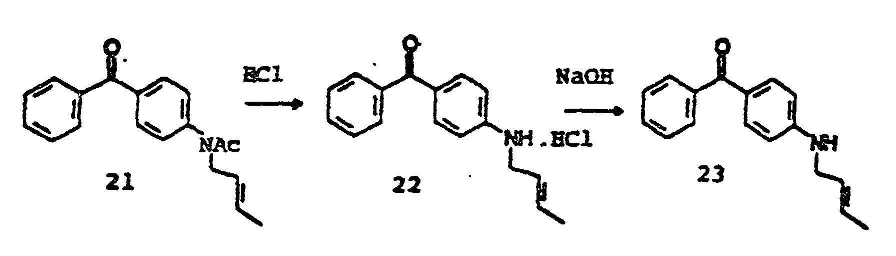 Compound **21** (8.6 g, 0.029 moles) was dissolved in 95% EtOH (15 ml), conc. HCl (15 ml) was added and the mixture was heated to ebullition for 3 hours. The TLC control (Hexane/EtOAc 1:1) evidenced the formation of a UV active spot with Rf = 0. **21** had not completely disappeared. EtOH was evaporated off, the acid solution was washed with EtOAc to remove the residual starting product, the solution was alkalinized with 5% NaOH and extracted again with EtOAc, dried over Na₂SO₄, filtered and the solvent was evaporated off. The resulting yellow oil was washed with Hexane/EtOAc 7:3, to precipitate compound **23** as a powdery yellowish solid (5.5 g, 0.022 moles). Hexane/EtOAc 7:3 Rf = 0.64. 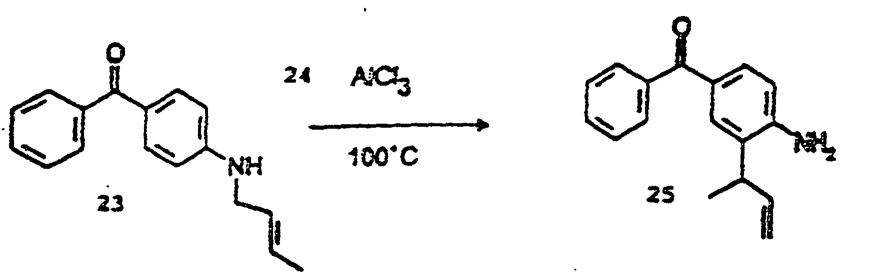 Compound **23** (0.2 g, 0.8 mmoles) was dissolved in toluene, **24** (0.1 g, 0.8 mmoles) was added and temperature was raised to 100°C for 5 hours. After a night at room temperature, heating was maintained for a further 5 hours. TLC (Hexane/EtOAc 8:2) showed no increases in the desired product. The mixture was left to cool, poured into 2N HCl (5 ml), alkalinized with 2N NaOH and extracted with EtOAc. The extracts were dried over Na₂SO₄ and filtered. The solvent was evaporated off. A crude of about 200 mg was obtained, 150 mg of which were purified on chromatographic column (Hexane: EtOAc 9:1). 18 mg of **25** were obtained (0.07 mmoles). Hexane: EtOAc 8:2 Rf = 0.4 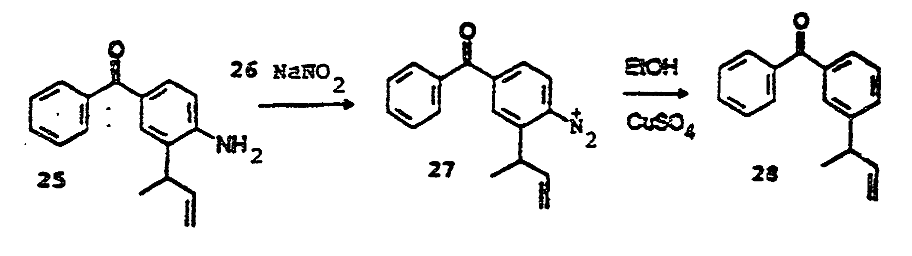 Compound **25** (30 mg, 0.12 mmoles) was dissolved in 4 ml of H₂O added with 15 µl of conc. HCl. The mixture was boiled for 5 minutes, then cooled on ice to 15°C and added with a further 15 µl of conc. HCl. After stirring for 15 minutes at 10°C, 8.8 mg of **26** (0.127 mmoles) dissolved in 0.4 ml of H₂O were added, stirring for 20 minutes. EtOH (3 ml) added with a spatula tip of CuSO₄ was heated to 60°C in another flask. This solution was added with the mixture containing the diazonium salt in small amounts (slight gas evolution), keeping for 30 minutes at 60°C. EtOH was evaporated off, the residue was extracted with EtOAc and dried over Na₂SO₄. The solvent was evaporated off. The crude (30 mg) was purified on preparative TLC Hexane: EtOAc 8:2. 10 mg of compound **28** (pale yellow oil) were obtained. Hexane:EtOAc 8:2 Rf = 0.72.

### EXAMPLE 3

### 4-(perfluoro-1-butanesulfonate)-3-(1'-methyl-2'-propen-1-yl)benzophenone.

To a solution of 3-(1'-methyl-2'-propen-1-yl)-4-hydroxybenzophenone (5 g, 0.02 mol) in acetone (25 ml), potassium carbonate (5.5 g, 0.04 mol) and the mixture was left stirring at room temperature for 15'. Then perfluoro-1-butanesulfonyl fluoride (6.6 g, 0.021 mol) was dropped and the solution refluxed for 3 hours. After cooling the inorganic salts were filtered off and the filtrate evaporated under vacuum to give a residue that was diluted with EtOAc (50 ml) and washed with brine (2x50 ml). After drying over Na₂SO₄ the solvent was evaporated to give 4-(perfluoro-1-butanesulfonate)-3-(1'-methyl-2'-propen-1-yl) benzophenone as yellowish oil (10.15 g, 0.019 mol). Yield 95%.
TLC (n-Hexane:EtOAc 9:1) Rf= 0.43.
¹H-NMR (CDCl₃) d 7.9-7.82 (dd, 3H, J= 8 Hz), 7.75-7.65 (m, 2H), 7.49 (t, 2H, J= 8Hz), 7.35 (d, 1H, J=8Hz), 6.05-5.92 (m, 1H), 5.21-5.03 (m, 2H), 3.95 (m, 1H), 1.41 (d, 3H, J= 8Hz).

### EXAMPLE 4

### 3-(1'-methyl-2'-propen-1-yl)benzophenone

To a solution of 4-(perfluoro-1-butanesulfonate)-3-(1'-methyl-2'-propen-1-yl)-benzophenone (6.13 g, 0.0115 mol) in dioxane (50 ml), triethylamine (4.96 ml, 0.036 mol) was added and then, under Ar, palladium acetate (0.108 g, 4.8x10⁻⁴ mol) and triphenylphosphine (0.25 g, 9.6x10⁻⁴ mol). Formic acid (1.1 g, 0.024 mol) was then added portionwise and the mixture was warmed at 60°C and left at the temperature for 1.5 h. After cooling the mixture was evaporated under vacuum; the residue was diluted with diethyl ether (50 ml), the formed precipitate was filtered off and the filtrate washed with 2N HCl (2x15 ml), 1N NaOH (2x8 ml), dried over Na₂SO₄ and evaporated under vacuum to give pure 3-(1'-methyl-2'-propen-1-yl)benzophenone (2.6 g 0.011 mol) as yellow oil. Yield =95%

Perfluorobutanesulfonic acid (3.21 g, 0.0107 mol) was recovered from basic aqueous layer (1N NaOH) by acidification (conc. HCl) and extraction with ethyl acetate (2x 20 ml). Recovering yield= 93%.
TLC (n-Hexane/EtOAc 9:1) Rf=0.8
¹H-NMR (CDCl₃) d 7.79-7.71 (d, 3H), 7.70-7.65 (m, 2H), 7.55-7.35 (m, 4H), 6.09-5.89 (m, 1H), 5.15-5.02 (m, 2H), 3.55 (m, 1H), 1.42 (d, 3H, J= 8Hz).

### EXAMPLE 5

### 4-(benzenesulfonate)-3-(1'-methyl-2'-propen-1-yl)benzophenone.

To a solution of 3-(1'-methyl-2'-propen-1-yl)-4-hydroxybenzophenone (0.9 g, 0.0036 mol) in acetone (10 ml), potassium carbonate (0.99 g, 0.0076 mol) was added and the mixture was left stirring at room temperature for 15'. Then benzenesulfonyl chloride (0.633 g, 0.0036 mol) was dropped and the solution was stirred at room temperature for 3 hours and then refluxed for 1 hour. After cooling inorganic salts were filtered off and the filtrate evaporated under vacuum to give a residue that was diluted with EtOAc (10 ml) and washed with brine (2x10 ml). After drying over Na₂SO₄ the solvent was evaporated to give 4-(benzenesulfonate)-3-(1'-methyl-2'-propen-1-yl)benzophenone as yellowish oil (1.4 g, 0.0036 mol).
Quantitative yield.
TLC (n-Hexane:EtOAc 9:1) Rf= 0.4.
¹H-NMR (CDCl₃) d 7.79-7.71 (dd, 2H, J=8Hz), 7.70-7.4 (m, 10H), 7.25 (d, 1H), 5.9-5.72 (m, 1H), 5.15-4.9 (m, 2H), 3.65 (m, 1H), 1.15 (d, 3H, J= 8Hz).

### EXAMPLE 6

### 3- (1'-methyl-2'-propen-1-yl)benzophenone

To a solution of 4-(benzenesulfonate)-3-(1'-methyl-2'-propen-1-yl)-benzophenone (0.31 g, 0.0008 mol) in DMF (5.5 ml), triethylamine (0.325 mg, 0.0032 mol) was added and then, under Ar, palladium acetate (9x10⁻³ g, 4x10⁻⁵ mol) and 1,3-Bis(diphenylphosphino)propane (1.8x10⁻⁴ g, 4.4x10⁻⁵ mol). Formic acid (0.147 g, 0.0032 mol) was then added portionwise and the mixture was warmed at 90°C and left at the temperature for 3 h. After cooling the mixture was poured in 2N HCl (10 ml) and extracted with EtOAc (2x10 ml). The organic layer was washed with brine (10 ml), dried over Na₂SO₄ and evaporated under vacuum to give a crude oil (0.21 g). Pure 3-(1'-methyl-2'-propen-1-yl)benzophenone (0.085 g 3.5x10⁻⁴ mol) was obtained as colourless oil by chromatographic purification (silica gel; n-Hexane/EtOAc 8:2). Yield =44%

The saturated compound was isolated in traces as side product.
TLC (n-Hexane/EtOAc 9:1) Rf=0.8
¹H-NMR (CDCl₃) d 7.79-7.71 (d, 3H), 7.70-7.65 (m, 2H), 7.55-7.35 (m, 4H), 6.09-5.89 (m, 1H), 5.15-5.02 (m, 2H), 3.55 (m, 1H), 1.42 (d, 3H, J = 8Hz).

### EXAMPLE 7

### 2-(2'-benzenesulfonate-5'-benzoylphenyl)propionic acid

To a solution of 4-(benzenesulfonate)-3-(1'-methyl-2'-propen-1-yl)-benzophenone (1 g, 2.55x10⁻³ mol) in CH₂Cl₂ (15 ml) an equal volume of H₂O, glacial acetic acid (0.3 ml), conc. H₂SO₄ (0.8 ml) and a catalytic amount of Aliquat 336 were added. Then potassium permanganate (1.2 g, 7.6x10⁻³ mol) was added to the mixture portionwise. The mixture was stirred 24 hours at room temperature until disappearence of starting material (TLC). An aqueous solution of sodium metabisulfite was added under stirring until disappearence of the dark brown colour of the solution. CH₂Cl₂ (5 ml) was added and the two phases separated; the organic layer was washed with water (2x 10 ml), dried over Na₂SO₄ and evaporated under vacuum to give a crude residue (1.05 g). The pure 2-(2'-benzenesulfonate-3'-benzoylphenyl)propionic acid (0.8 g, 1.9x10⁻³) was obtained by chromatographic purification (silica gel, CH₂Cl₂/MeOH 95:5). Yield 76%.
TLC (CH₂Cl₂/MeOH 9:1) Rf=0.45
¹H-NMR (CDCl₃) d 8.0-7.15 (m, 13H), 4.15-3.95 (q, 1H,J=7Hz), 1.35 (d, 3H, J= 7Hz).

### EXAMPLE 8

### 2-(2'-perfluorobutanesulfonate-5'-benzoylphenyl)propionic acid

To a solution of 4-(perfluorobutanesulfonate)-3-(1'-methyl-2'-propen-1-yl)benzophenone (0.2 g.,0.37x10⁻³ mol) in CH₂Cl₂ (2.5 ml) an equal volume of H₂O, glacial acetic acid (0.046 ml), conc. H₂SO₄ (0.12 ml) and a catalytic amount of Aliquat 336 were added. Then potassium permanganate (0.173 g, 1.09x10⁻³ mol) was added to the mixture portionwise. The mixture was stirred 24 hours at room temperature until disappearence of starting material (TLC). An aqueous solution of sodium metabisulfite (0.115 g, 1 ml) was added under stirring until disappearence of the dark brown colour of the solution. CH₂Cl₂ (3 ml) was added and the two phases were separated; the organic layer was washed with water (2x 5 ml), dried over Na₂SO₄ and evaporated under vacuum to give a crude residue (0.25 g). 2-(2'-Perfluorobutanesulfonate-5'-benzoylphenyl)propionic acid (0.17 g, 0.3x10⁻³ mol) was obtained, by chromatographic purification (silica gel, CH₂Cl₂/MeOH 95:5). Yield 83%. The isolated product was contaminated by traces of Aliquat 336 (4%, NMR).
TLC (CH₂Cl₂/MeOH 9:1) Rf=0.5.
¹H-NMR (CDCl₃) d 8.0-7.41 (m, 8H), 4.25-4.1 (q, 1H,J=7Hz), 1.55 (d, 3H, J= 7Hz).

### EXAMPLE 9

Methyl esters of 2-(2'-benzenesulfonate-3'-benzoylphenyl)propionic acid and 2-(2'-perfluorobutane sulfonate -3'-benzoylphenyl) propionic acid were easily obtained starting from the acids according to the usual esterification methods (diazomethane/ether; dry MeOH/ and catalytic amounts of H₂SO₄, benzensulfonic or p-toluensulfonic acids).

By hydrogenolysis of the following esters: 2-(2'-perfluorobutane sulfonate-5'-benzoylphenyl) propionic acid methyl ester.
¹H-NMR (CDCl₃) Ù 7.8-7.4 (m, 8H), 4.25-4.1 (q, 1H,J=7Hz), 3.95 (s,3H) 1.55 (d, 3H, J= 7Hz).
2-(2'-benzenesulfonate-5'-benzoylphenyl)propionic acid methyl ester.
¹H-NMR (CDCl₃) Ù 8.1-7.9 (d, 1H),7.9-7.5 (m, 11H), 7.33 (d,1H J=8Hz), 4.15-3.95 (q, 1H, J=7Hz),3.75 (s, 3H), 1.35 (d, 3H, J= 7Hz).

The 2-(3'-benzoylphenyl)propionic acid methyl ester (methyl ketoprofenate) was obtained according the following procedure.

To a solution of 2-(2'-perfluorobutane sulfonate -5'-benzoylphenyl) propionic acid methyl ester (6.8 g, 0.012 mol) in dioxane (50 ml), triethylamine (4.96 mL, 0.036 mol) was added and then, under Ar, palladium acetate (0.108 g, 4.8x10⁻⁴ mol) and triphenylphosphine (0.25 g, 9.6x10⁻⁴ mol). Formic acid (1.1 g, 0.024 mol) was then added portionwise and the mixture was warmed at T=60 °C for 1.5 h. After cooling the mixture was evaporated under vacuum; the residue was diluted with diethyl ether (50 ml), the formed precipitate was filtered off. The filtrate was washed with 2N HCl (2x15 ml), 1N NaOH (2x8 mL), dried over Na₂SO₄ and evaporated under vacuum to give pure 2-(3'-benzoylphenyl)propionic acid methyl ester (2.14 g 0.008 mol) as yellow oil. Yield =67%; TLC (n-Hexane/EtOAc 9:1) Rf=0.53. ¹H-NMR (CDCl₃) Ù 7.85-7.3 (m, 9H), 3.85 (q, 1H, J=8Hz), 3.65 (s, 3H), 1.5 (d, 3H, J= 8Hz).

Perfluoro-1-butansulfonic acid (3.21 g, 0.0107 mol) was recovered from basic aqueous layer (NaOH 1N) by acidification (HCl conc.) and extraction with ethyl acetate (2x 20 ml).

## Claims

1. A process for the preparation of 2-(3-benzoyl-phenyl)-propionic acid which comprises:
a) Claisen rearrangement of compounds of formula (I) : 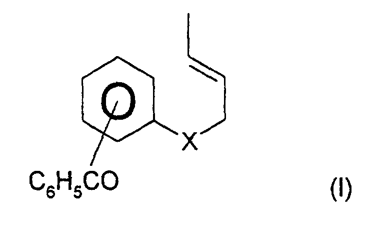 in which X is O, S or NH, the benzoyl group is at the ortho or para position to the X-alkylene group, provided that where X is S, the reaction is carried out in the presence of C3-C10 fatty acid anhydrides, to give a compound of formula (II) 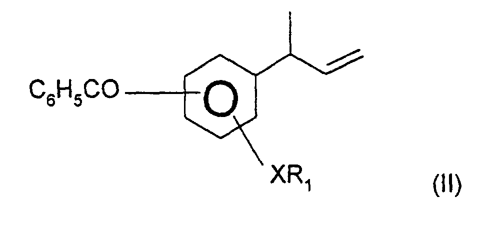 wherein XR₁ is at the ortho position to the allyl chain and is a OH, NH₂, SH or S(C₃-C₁₀) acyl group;
b) for compounds where X is S, desulfuration with Raney nickel,
for compounds where X is NH, optional deamination via nitrosation with alkali nitrites followed by decomposition of the diazonium salt in the presence of copper sulfate catalytic amounts,
for compounds where X is O, optional dehydroxylation by forming a trifluoromesylate which is then treated with formic acid and triethylamine in the presence of palladium acetate/triphenylphosphine complex;
c) oxidative cleavage of the compound (II);
d) dehydroxylation or deamination of the product obtained from (c), when such reactions are not already carried out in step b).

2. A process according to claim 1, wherein compounds of formula II in which X is 0 are used.

3. A process according to claim 1, wherein compounds of formula II in which X is NH are used.

4. A process according to claim 1, wherein compounds of formula II in which X is S are used.

5. A process according to any one of the above claims, wherein step c) is effected with KMnO₄ in phase transfer conditions.

## Patentansprüche

1. Verfahren für die Herstellung von 2-(3-Benzoyl-phenyl)propionsäure, das umfaßt:
a) die Clalsen-Umlagerung der Verbindungen der Formel (I): 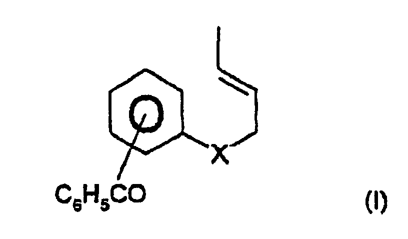 worin X O, S oder NH ist, die Benzoylgruppe In der ortho- oder para-Position zur X-Alkylengruppe steht, vorausgesetzt dass X S ist, und die Reaktion in Gegenwart von C₃-C₁₀-Fettsäureanhydriden durchgeführt wird, um eine Verbindung der Formel (II) zu ergeben 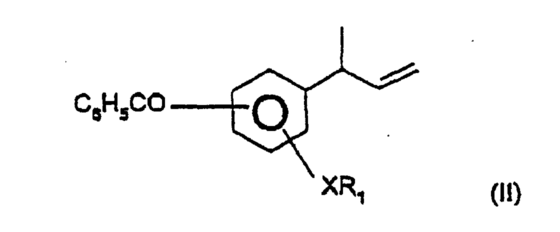 worin XR₁ in der ortho-Position zur Allylkette steht und eine OH-, NH₂-, SH- oder S(C₃-C₁₀)Acylgruppe ist;
b) für Verbindungen, worin X S ist, die Desulfurierung mit Raneynickel, für Verbindungen, worin X NH ist, wahlweise die Deaminlerung über die Nitrosierung mit Alkalinitriten, gefolgt durch die Zersetzung des Dlazonlumsalzes in Gegenwart von katalytischen Mengen von Kupfersulfat, für Verbindungen, worin X O ist, wahlweise die Dehydroxylierung durch Bildung eines Trifluormesylates, das dann mit Ameisensäure und Triethylamin in Gegenwart eines Palladiumacetat/Triphenylphosphin-Komplexes behandelt wird;
c) die oxidative Spaltung der Verbindung (II);
d) die Dehydroxylierung oder Deaminierung des Produktes erhalten aus (c), wenn diese Reaktionen nicht bereits in Schritt (b) durchgeführt wurden.

2. Verfahren nach Anspruch 1, worin die Verbindungen der Formel II, worin X O ist, verwendet werden.

3. Verfahren nach Anspruch 1, worin die Verbindungen der Formel II, worin X NH ist, verwendet werden.

4. Verfahren nach Anspruch 1, worin die Verbindungen der Formel II, worin X S ist, verwendet werden.

5. Verfahren nach irgendeinem der obigen Ansprüche, worin der Schritt (c) mit KMnO₄ unter Phasentransferbedingungen durchgeführt wird.

## Revendications

1. Procédé pour la préparation d'acide 2-(3-benzoyl-phényl)-propionique, qui comprend :
a) la transformation de Claisen de composés de formule (I) : 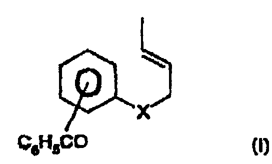 dans laquelle X est O, S ou NH, le groupe benzoyle est en position ortho ou para par rapport au groupe X-alkylène, étant entendu que lorsque X est S, la réaction est effectuée en présence d'anhydrides d'acide gras en C₃ à C₁₀, pour donner un composé de formule (II) 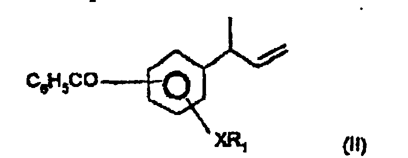 dans laquelle XR₁ est en position ortho par rapport à la chaîne allyle et est un groupe OH, NH₂, SH ou S-acyle en C₃ à C₁₀ ;
b) pour les composés dans lesquels X est S, une désulfuration avec du nickel de Raney,
pour les composés dans lesquels X est NH, une désamination facultative par nitrosation avec des nitrites alcalins suivie par une décomposition du sel de diazonium en présence de quantités catalytiques de sulfate de cuivre,
pour les composés dans lesquels X est O, une déshydroxylation facultative en formant un trifluoromésylate qui est ensuite traité avec de l'acide formique et de la triéthylamine en présence d'un complexe acétate de palladium/triphénylphosphine ;
c) le clivage oxydatif du composé (II) ;
d) la déshydroxylation ou désamination du produit obtenu en (c), lorsque de telles réactions ne sont pas déjà effectuées dans l'étape b).

2. Procédé selon la revendication 1, dans lequel des composés de formule II dans lesquels X est O sont utilisés.

3. Procédé selon la revendication 1, dans lequel des composés de formule II dans lesquels X est NH sont utilisés.

4. Procédé selon la revendication 1, dans lequel des composés de formule II dans lesquels X est S sont utilisés.

5. Procédé selon l'une quelconque des revendications ci-dessus, dans lequel l'étape c) est effectuée avec KMnO₄ dans des conditions de transfert de phase.
